# EUROPÄISCHE PATENTSCHRIFT

(11) **EP 1 537 210 B1**
(45) Veröffentlichungstag und Bekanntmachung des Hinweises auf die Patenterteilung: **31.05.2006**
(21) Anmeldenummer: 03793596.2
(22) Anmeldetag: 02.09.2003
(51) Int. Cl.: C12N 15/11, C12N 15/09, C07H 21/00, A61K 31/70

(54) **DECOY-OLIGONUKLEOTID-HEMMUNG DER CD40-EXPRESSION**
DECOY-OLIGONUCLEOTIDE-INHBITION OF CD40-EXPRESSION
INHIBITION D'OLIGONUCLEOTIDE-LEURRE D'EXPRESSION CD40

(30) Priorität: 02.09.2002 DE 10240417
(43) Veröffentlichungstag der Anmeldung: 08.06.2005
(73) Patentinhaber: Avontec GmbH, 37073 Göttingen (DE)
(72) Erfinder: HECKER, Markus, 37075 Göttingen (DE); WAGNER, Andreas, H., 37083 Göttingen (DE)
(74) Vertreter: Dehmel, Albrecht
(86) Internationale Anmeldenummer: PCT/DE2003/002900
(87) Internationale Veröffentlichungsnummer: WO 2004/022749

(56) Entgegenhaltungen:
- WO-A-95/11687
- DATABASE WPI Derwent Publications Ltd., London, GB; AN 2001-316865 XP002268675 HECKER M. ET AL.: "Modulating gene transcription in an endothelial, vascular or cardiac cell, useful for treating vascular proliferative disease comprises contacting the cell with a nucleic acid capable of sequence-specific binding to AP-1 or C/EBP." & JP 2001 095573 A (AVONTEC GMBH), 10. April 2001 (2001-04-10)

## Beschreibung

Die vorliegende Erfindung betrifft Decoy-Oligonukleotide mit der Nukleinsäuresequenz gemäß SEQ ID NO:1 bis 36 sowie deren Verwendung als Arzneimittel.

Die Transplantation solider Organe stellt in der Regel die *ultima ratio* in der Therapie von Erkrankungen dar, bei denen das zu ersetzende Organ im Empfängerorganismus schwer geschädigt ist bzw. seine Funktion nicht mehr in ausreichender Weise erfüllen kann. Dies ist beispielsweise im Terminalstadium der Herzinsuffizienz der Fall, aber auch beim akuten oder chronischen Nieren- oder Leberversagen. Routinemäßig aber weniger häufig transplantiert werden neben den genannten Organen auch Pankreas, Lunge und Dünndarm. Auch eine kombinierte Transplantation mehrerer Organe ist möglich. Zusätzlich zu den soliden Organen werden auch die Hornhaut der Augen sowie hämapoietische Stammzellen aus dem Knochenmark transplantiert.

In Deutschland wurden im Jahr 2000 insgesamt 3130 Transplantationen solider Organe durchgeführt (Eurotransplant), wobei das nach wie vor größte Problem die akute Abstoßung des Spenderorgans durch den Empfangerorganismus darstellt. Diese Abstoßungsreaktion (*hostversus-graft reaction)* ist umso ausgeprägter, je weniger die immunologischen Merkmale des Spenders mit denen des Empfängers übereinstimmen (mangelnde Histokompatibilität). Zwar gelingt es in der Regel bei ausreichender Histokompatibilität die Abstoßungsreaktion durch entsprechende Medikamente (Immunsuppressiva) zu unterdrücken, die Dauerbehandlung mit diesen Medikamenten kann aber schwere Nebenwirkungen hervorrufen. So entwickeln die transplantierten Patienten infolge ihrer eingeschränkten Immunabwehr häufig Tumoren und Infektionen; zudem wird die chronische Abstoßung der transplantierten Organe verstärkt. Bei dieser auch als Vaskulopathie bezeichneten Degeneration der das transplantierte Organ versorgenden Arterien und Arteriolen handelt es sich um eine beschleunigte Sonderform der Atherosklerose (Transplantat-Atherosklerose), die über eine fortschreitende Funktionseinschränkung sukzessive zum Versagen des transplantierten Organs führt. Falls eine Retransplantation (z. B. mangels Organverfügbarkeit) nicht möglich ist, hat die chronische Transplantatabstoßung insofern unweigerlich den Tod des betroffenen Patienten zur Folge.

Immunologisch betrachtet stellt die akute Abstoßung transplantierter Organe (nicht zu verwechseln mit der wesentlich selteneren, hyperakuten Abstoßungsreaktion, die Antikörper-vermittelt ist) eine Überempfindlichkeitsreaktion vom Typ IV (verzögerter Reaktionstyp oder *delayed type hypersensitivity*) dar. Das Antigen (in der Regel Histokompatibilitätsantigene auf den die Blutgefäße des Spenderorgans auskleidenden Endothelzellen) wird von (Gewebe)Makrophagen phagozytiert, prozessiert und naiven T-Helferzellen (CD4-positiv) präsentiert; die Sensibilisierung der T-Helferzellen dauert mehrere Tage. Beim Zweitkontakt wandeln sich die so sensibilisierten T-Helferzellen in Th1-Zellen um; dabei spielt die CD154-Ligand-vermittelte Kostimulation der Antigen-präsentierenden Zelle (diese exprimiert den entsprechenden CD40-Rezeptor) eine wichtige Rolle, da es über diesen Signalweg zur Freisetzung von Interleukin-12 aus den Makrophagen kommt. Interleukin-12 leitet die Differenzierung und Proliferation der T-Helferzellen ein. Die Th1-Zellen ihrerseits regen über bestimmte Wachstumsfaktoren (z.B. *granulocytemacrophage colony-stimulating factor)* die Monozytenbildung im Knochenmark an, rekrutieren diese mit Hilfe bestimmter Chemokine (z.B. *macrophage migration inhibitory factor* [MIF]) und aktivieren sie über die Freisetzung von Interferon-γ. Die daraus resultierende sehr starke Entzündungsreaktion kann das transplantierte Gewebe in großem Umfang zerstören. Bei der Transplantatabstoßung sind darüber hinaus CD8-positive zytotoxische T-Zellen beteiligt, die ihre Zielzellen durch Zytolyse und/oder Induktion des programmierten Zelltods zerstören. Wie die CD4-positiven Th1-Zellen sind zytotoxischen T-Zellen erst durch vorherige Antigenpräsentation in der Lage sind, ihr Ziel (die fremde Zelloberfläche) zu erkennen und sich entsprechend zu "bewaffnen". Hierbei ist die CD154-CD40-vermittelte Kostimulation ebenfalls bedeutsam. Neuesten Erkenntnissen zufolge haben die Endothelzellen des Spenderorgans möglicherweise selbst Antigenpräsentierende und kostimulatorische Eigenschaften.

Mit der Transplantatabstoßung vergleichbar, allerdings im umgekehrten Sinne, ist die *graft-versus-host disease* (GvHD), die im Zuge von allogenen Knochenmarkstransplantationen (zwischen genetisch nicht identischen Individuen) bei circa 40% der Empfänger auftritt. In der bis zu dreimonatigen Akutphase greifen die mit den Stammzellen übertragenen T-Zellen des Spenders den Wirtsorganismus an, die resultierende unter Umständen schwere Entzündungsreaktion manifestiert sich bevorzugt in der Haut, seltener im Gastrointestinaltrakt und in der Leber. Insofern ist bei diesen Patienten ebenfalls eine Immunsuppression indiziert mit den bereits beschriebenen potenziell ernsten Nebenwirkungen. Bei der Initiierung dieser Entzündungsreaktion sind wiederum Endothelzellen, diesmal die des Empfängerorganismus beteiligt.

Neben der akuten GvHD gibt es auch die chronische Verlaufsform, die einer längeren Immunsuppression bedarf.

Die zur Verhinderung der akuten Transplantatabstoßung eingesetzten Immunsuppressiva variieren in der Regel in Abhängigkeit vom Organtyp bzw. sind nur für die Immunsuppression nach der Transplantation bestimmter Organe zugelassen. Eine typische Therapie für Empfänger eines Herztransplantats stellt die Kombination von Cyclosporin A mit Azathioprin und Kortison dar, wobei in letzter Zeit Cyclosporin A zunehmend durch Tacrolimus und Azathioprin durch Mycophenolatmofetil ersetzt wird. Cyclosporin A, Rapamycin und Tacrolimus hemmen die T-Zellaktivierung, Azathioprin und Mycophenolatmofetil sind Antimetabolite und Corticosteroide wirken antientzündlich durch Hemmung der Genexpression. Trotz ihrer unumstrittenen therapeutischen Wirkung verläuft die lebenslange systemische Therapie mit diesen Medikamenten nicht ohne z. T. gravierende Nebenwirkungen. Hierzu zählen insbesondere Myelotoxizität, Neurotoxizität, Nephrotoxizität, Stoffwechselstörungen bis hin zur Induktion eines Diabetes mellitus, arterieller Hypertonus, Infektionen und Malignome. Auch die GvHD wird in der Regel mit den vorgenannten Medikamenten, häufig in Kombination, behandelt.

Antigenpräsentierende Zellen und T-Zellen kommunizieren u. a. über CD40-Rezeptor und GD154-Ligand und diese Kostimulation spielt eine wichtige Rolle bei der Th1-Zell-vermittelten Entzündungsreaktion bzw. der Aktivierung zytotoxischer T-Zellen im Rahmen der Transplantatabstoßung ebenso wie der GvHD. Wie Antigenpräsentierende Zellen exprimieren auch Endothelzellen konstitutiv den CD40-Rezeptor. Die Interaktion der Endothelzellen mit CD154-exprimierenden T-Helferzellen (naive T-Helferzellen und/oder aktivierte Th1-Zellen) resultiert in einer gesteigerten Chemokin- und Adhäsionsmolekülexpression dieser Zellen. Infolgedessen kommt es zu einer vermehrten Rekrutierung und Aktivierung zirkulierender Monozyten, diese emigrieren in die Gefäßwand und differenzieren sich zu Makrophagen. Darüber hinaus setzen die Endothelzellen, im Gegensatz zu anderen Antigen-präsentierenden Zellen, ausschließlich nach CD40-Aktivierung biologisch aktives Interleukin-12 frei. Interleukin-12 ist der wichtigste Faktor für die Differenzierung von naiven T-Helferzellen in Th1-Zellen und fördert die anschließende klonale Expansion (Proliferation) der Th1-Zellen. Die verstärkte Bildung von Interferon-γ durch die ausdifferenzierten Th1-Zellen stimuliert nicht nur die Aktivität der infiltrierten Makrophagen, sondern verstärkt auch die Expression von CD40 in den Endothelzellen. In der Folge kann sich ein Teufelskreis entwickeln, bei dem sich Endothelzellen, T-Helferzellen und Makrophagen gegenseitig stimulieren und so die das Transplantat (akute Transplantatabstoßung) bzw. den Empfängerorganismus (GvHD) schädigende Entzündungsreaktion aufrecht halten.

Insofern stellt die Blockade der CD154/CD40-vermittelten Kostimulation ein viel versprechendes Ziel für die Abschwächung bzw. Hemmung der akuten Transplantatsabstoßung dar. So gibt es tierexperimentelle Hinweise dahingehend, dass die Antikörper-gestützte Neutralisation von CD154 unmittelbar im Anschluss an die Transplantation sogar eine Immmtoleranz erzeugen kann. Zudem wird die chronische Transplantatsabstoßung (Transplantat-Atherosklerose) durch diese Intervention therapeutisch günstig beeinflusst. Ein Nachteil dieser Antikörpertherapie besteht u. a. in der Gefahr von Überempfindlichkeitsreaktionen (gegen den Antikörper), vor allem bei wiederholter Applikation, sowie die zumindest für gewebeständige Antigene (z.B. in die Wand der Blutgefäße des Spenderorgans emigrierte T-Zellen) schlechte Zugänglichkeit, da die Antikörper in der Regel von der Blutseite appliziert werden müssen und dann an der Endothelzellbarriere scheitern.

Der vorliegenden Erfindung liegt daher die Aufgabe zugrunde Mittel für eine Prävention und/oder Therapie der akuten und chronischen Transplantatabstoßung einschließlich der GvHD und den hiermit assoziierten Folgen für Morbidität und Mortalität der betroffenen Patienten zur Verfügung zu stellen. Die Aufgabe wird durch den in den Patentansprüchen definierten Gegenstand gelöst.

Die Erfindung wird durch die nachfolgenden Figuren näher erläutert:

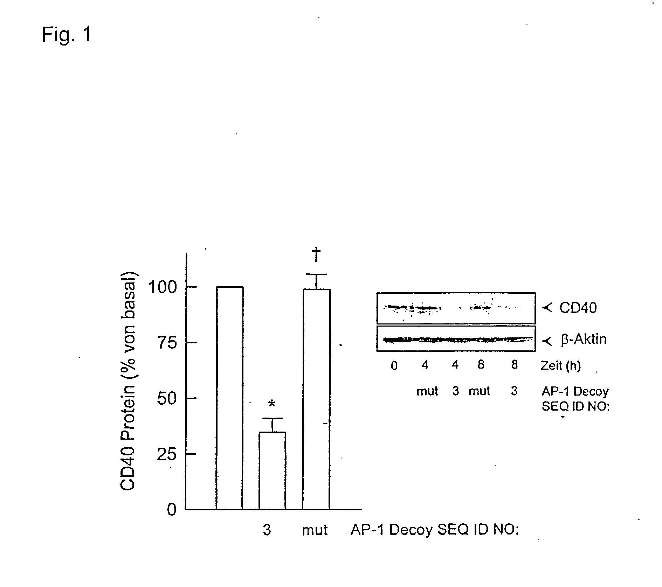

Figur 1 zeigt in einem Balkendiagramm die Auswirkungen eines AP-1 Cis-Element Decoys (SEQ ID NO: 3) und eines mutierten Kontroll-Oligonukleotids (mut) auf die basale CD40-Proteinexpression in ruhenden humanen Endothelzellen, die für 8 Stunden mit dem entsprechenden Oligonukleotid (10 µM) inkubiert wurden (n=7-10, statistische Zusammenfassung, bezogen in % auf die basale Expression, *P < 0,05 versus basal; ^{†}P < 0,05 versus Cis-Element-Decoy). Die repräsentative Western Blot-Analyse zeigt die Auswirkungen (4 bzw. 8 Stunden Inkubation) der verwendeten Nukleinsäuren auf den basalen CD40-Proteingehalt in ruhenden Zellen. β-Aktin (interner Standard) dient zum Nachweis, dass gleiche Proteinmengen analysiert wurden.

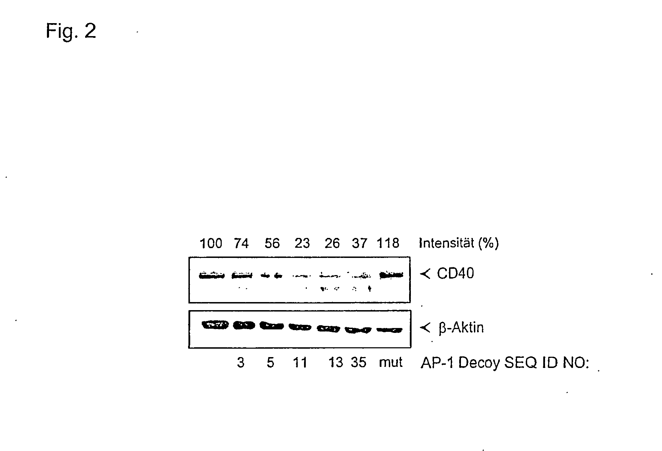

Figur 2 zeigt in einer repräsentativen Western Blot-Analyse die Effekte ausgewählter AP-1 Cis-Element Decoys (SEQ ID NO: 3, 5, 11, 13 und 35) und eines mutierten Kontroll-Oligonukleotids (mut) auf die basale CD40-Proteinexpression in ruhenden humanen Endothelzellen, die für 8 Stunden mit den entsprechenden Cis-Element Decoys (10 µM) inkubiert wurden. Die relativen Intensitäten (%), bestimmt durch densitometrische Auswertung (One-Dscan-Gel Analysis Software, Scanalytics, Billerica, MA, USA), sind bezogen auf den Maximalwert des CD40-Proteingehalts in Endothelzellen, die nicht mit einem AP-1 Cis-Element Decoy inkubiert wurden, angegeben.

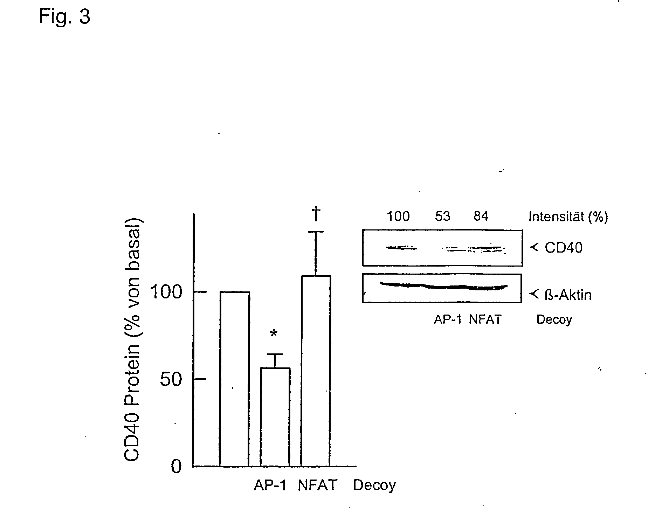

Figur 3 zeigt in einem Balkendiagramm und einer repräsentativen Western Blot-Analyse mit β-Aktin als internem Standard den Effekt des AP-1 Cis-Element Decoy SEQ ID NO: 3 im Vergleich zu der fehlenden Wirkung eines NFAT *(nuclear factor of activated T-cells)* Cis-Element Decoys auf die basale CD40-Proteinexpression in ruhenden humanen Endothelzellen, die für 8 Stunden mit den entsprechenden Oligonukleotid (10 µM) inkubiert wurden. Statistische Zusammenfassung (n=4, bezogen in % auf die basale Expression; *P < 0,05 versus basal; ^{†}P < 0.05 versus AP-1 Cis-Element Decoy).

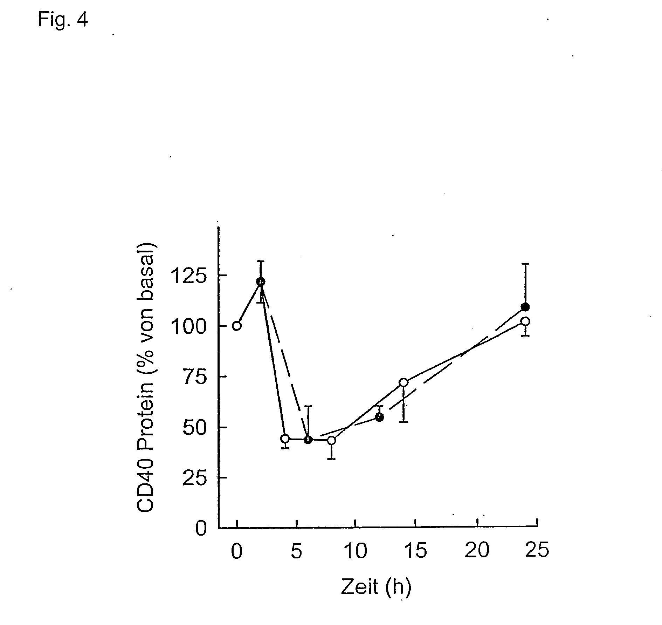

Figur 4 zeigt in einem Liniendiagramm den Effekt einer Dauerexposition (offene Kreise) bzw. einer zweistündigen Vorinkubation (gefüllte Kreise) mit dem AP-1 Cis-Element Decoy SEQ ID NO: 3 (10 µM) auf die basale CD40-Proteinexpression in ruhenden humanen Endothelzellen über einen Zeitraum von 24 Stunden (n=3-7).

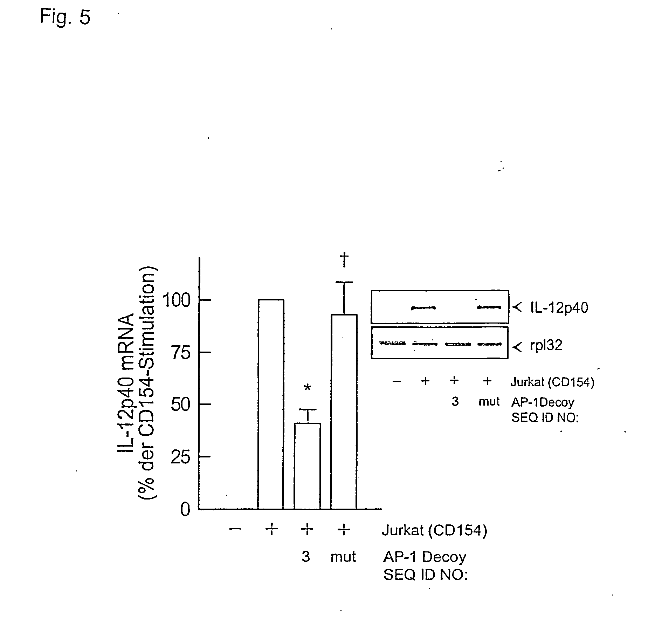

Figur 5 zeigt in einem Balkendiagramm und einer RT-PCR-Analyse die Auswirkungen einer Vorinkubation (4 Stunden, 10 µM) mit dem AP-1 Cis-Element Decoy SEQ ID NO: 3 bzw. eines mutierten Kontroll-Oligonukleotids (mut) auf die nachfolgende CD40 Ligand- (Exposition gegenüber CD40-Ligand-exprimierenden Jurkat T-Zellen; CD154) induzierte IL-12p40 mRNA Expression in humanen Endothelzellen (n=3). Repräsentative RT-PCR-Analyse und statistische Zusammenfassung (bezogen in % auf den Maximalwert der IL-12-p40 Expression bei CD154-Stimulation, *P < 0,05 versus CD154; ^{†}P < 0,05 versus AP-1 Cis-Element Decoy).

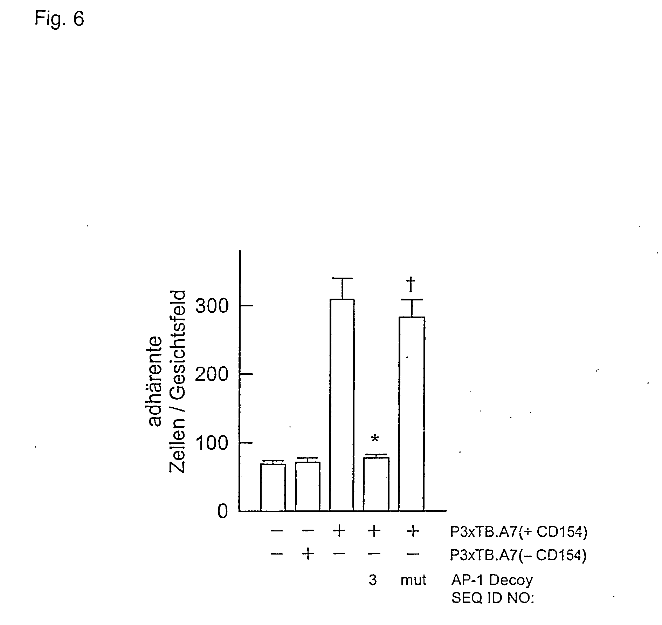

Figur 6 zeigt in einem Balkendiagramm die Wirkung des AP-1 Cis-Element Decoy SEQ ID NO: 3 im Vergleich zu dem Kontroll-Oligonukleotid (mut) auf die Adhäsion von humanen THP-1 Monozyten an humanen Endothelzellen, die 8 Stunden mit dem entsprechenden Oligonukleotid (10 µM) vorinkubiert und anschließend für 12 Stunden mit humanem CD154-transfizierten Maus-Myelomzellen (P3xTB.A7, CD154) ko-kultiviert wurden (statistische Zusammenfassung, n=10-13, *P < 0,05 versus CD154; ^{†}P < 0.05 versus AP-1 Cis-Element Decoy). Nicht transfizierte P3xTB.A7-Zellen (-CD154) wurden als Negativkontrolle mitgeführt. Vor Beginn der THP-1-Zell-Perfusion wurden die Myelomzellen von den Endothelzellen durch einen Waschschritt mit Medium nahezu vollständig entfernt.

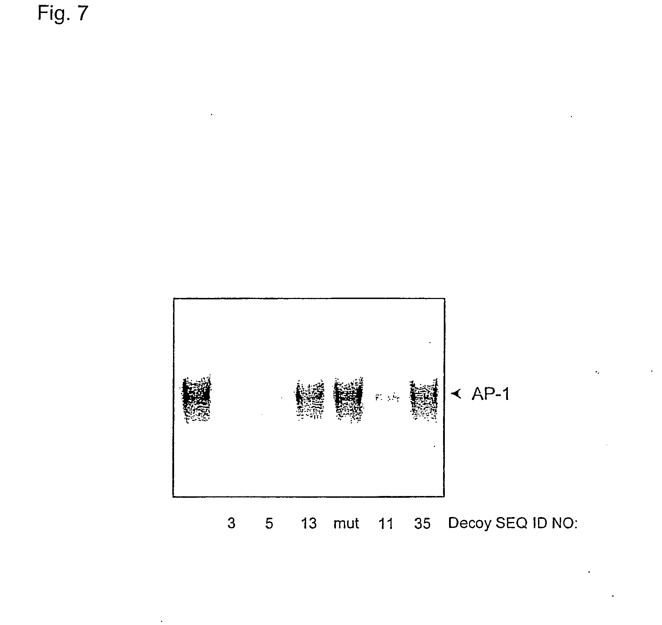

Figur 7 zeigt in einer repräsentativen elektrophoretischen Mobilitäts-Shift-Analyse (EMSA) die Wirkung eines 50-fachen Überschusses von ausgewählten AP-1 Cis-Element Decoys (SEQ ID NO: 3, 5, 11 13 und 35) im Vergleich zu einem mutierten Kontroll-Oligonukleotid (mut) auf die Bildung von DNA-Protein-Komplexen zwischen einem ³²P-markierten Oligonukleotid (11 fmol), das spezifisch den Transkriptionsfaktor AP-1 bindet, und einer Kemproteinpräparation aus humanen THP-1 Monozyten in einen 15 µl Reaktionsansatz.

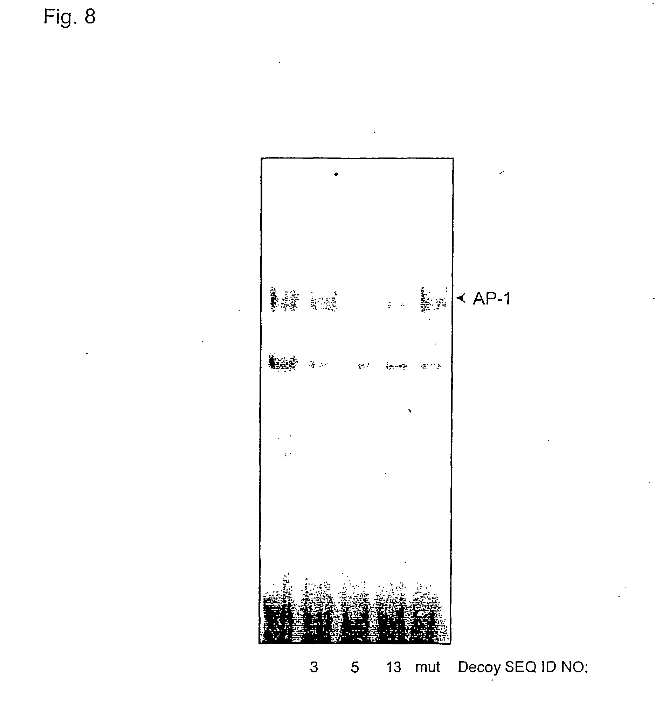

Figur 8 zeigt in einer repräsentativen EMSA den Effekt ausgewählter AP-1 Cis-Element Decoys (SEQ ID NO: 3, 5 und 13) und eines mutierten Kontroll-Oligonukleotids (mut) auf die Translokation von AP-1 in den Kern humaner Endothelzellen, die 4 Stunden mit den entsprechenden Cis-Element Decoy (10 µM) inkubiert wurden. Repräsentative EMSA, welche die zelluläre Aufnahme (und Wirkung) der verschiedenen Cis-Element Decoys in humanen Endothelzellen bestätigt. Vergleichbare Ergebnisse wurden in mindestens zwei weiteren unabhängigen Experimenten erhalten.

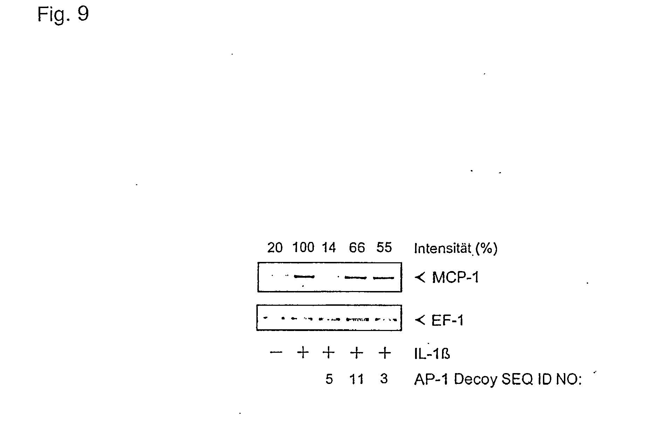

Figur 9 zeigt in einer repräsentativen RT-PCR die Auswirkungen einer Vorinkubation (4 Stunden, 10 µM) mit ausgewählten AP-1 Cis-Element Decoys (SEQ ID NO: 3, 5 und 11) auf die MCP-1 (*monocyte chemoattractant protein-1*) Expression in humanen Endothelzellen, die für 6 Stunden mit 60 U/ml Interleukin-1β (IL-1β) inkubiert wurden. Repräsentative RT-PCR (die relativen Intensitäten (%), bestimmt durch densitometrische Auswertung, sind bezogen auf den Maximalwert bei IL-1β-Stimulation angegeben).

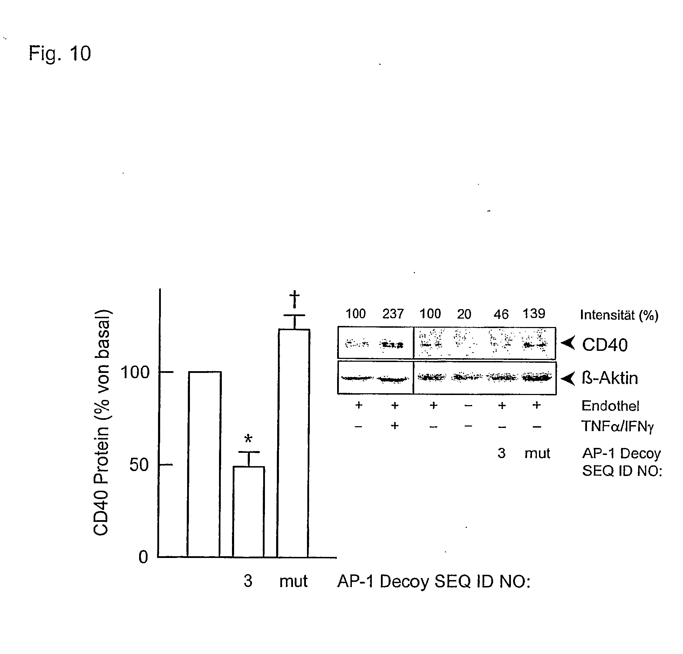

Abbildung 10 zeigt in einem Balkendiagramm und einer repräsentativen Western Blot-Analyse den Effekt eines AP-1 Cis-Element Decoys (SEQ ID NO: 3) und eines mutierten Kontroll-Oligonukleotids (mut) auf die basale CD40-Proteinexpression in isolierten endothelintakten Segmenten der Aorta der Ratte, die in Waymouth-Medium inkubiert wurden. Nach 1 Stunde Vorinkubation wurden die Cis-Element Decoys (10 µM) dem Inkubationsmedium zugesetzt und für 11 Stunden mit den Gefäßsegmenten inkubiert (6-8 Segmente von 5 verschiedenen Tieren, statistische Zusammenfassung, bezogen in % auf die basale Expression, *P < 0,05 versus basal; ^{†}P < 0.,05 versus Cis-Element-Decoy). Die repräsentative Western Blot-Analyse zeigt exemplarisch, dass das CD40-Protein in den untersuchten Gefäßsegmenten primär in den Endothelzellen lokalisiert ist und dessen Expression durch die Zugabe der Zytokine Tumornekrosefaktor-α (TNFα, 1000 U/ml) und Interferon-γ (IFNγ, 100 U/ml) zum Inkubationsmedium für 12 Stunden deutlich gesteigert werden kann. Die Detektion von β-Aktin (interner Standard) dient zum Nachweis, dass gleiche Proteinmengen analysiert wurden.

Der hier verwendete Ausdruck "Decoy-Oligonukleotid" oder "Cis-Element Decoy" bezeichnet ein doppelsträngiges DNA-Molekül, das eine Sequenz aufweist, an die der Transkriptionsfaktor AP-1 in der Zelle bindet, und die der natürlichen AP-1 Kernbindungssequenz im Genom entspricht oder ähnelt (Derivat). Das Cis-Element Decoy wirkt somit als Molekül zur kompetitiven Inhibierung (Neutralisierung) von AP-1.

Transkriptionsfaktoren sind DNA-bindende Proteine, die sich im Zellkern an die Promotorregion eines oder mehrerer Gene anlagern und dadurch deren Expression, d.h. die Neubildung der Proteine für welche diese Gene kodieren, steuern. Neben der physiologisch wichtigen Kontrolle von Entwicklungs- und Differenzierungsprozessen im menschlichen Körper haben Transkriptionsfaktoren, vor allem wenn sie die Genexpression zum falschen Zeitpunkt aktivieren, ein hohes Krankheitspotenzial. Zusätzlich können (unter Umständen dieselben) Transkriptionsfaktoren Gene mit schützender Funktion blockieren und prädisponierend für die Ausbildung einer Erkrankung wirken.

Die vorliegende Erfindung besteht daher in der Bereitstellung eines Decoy-Oligonukleotids, das in der Lage ist, sequenzspezifisch an den Transkriptionsfaktor *activator* oder *activating protein-1* (AP-1) zu binden und eine der folgenden Sequenzen hat, wobei hier nur jeweils ein Strang des Decoy-Oligonukleotids wiedergegeben ist und der komplementäre Strang ebenfalls umfasst ist:
5'-VTGAGTCAS-3' (SEQ ID NO:1), wobei V = A, C oder G und S = C oder G bedeutet
5'-STGACTCAB-3' (SEQ ID NO:2), wobei S = C oder G und B= G, C oder T bedeutet
5'-CGCTTGATGACTCAGCCGGAA-3' (SEQ ID NO:3),
5'-GTGCTGACTCAGCAC-3' (SEQ ID NO:5),
5'-GTGGTGACTCACCAC-3' (SEQ ID NO:7),
5'-AGTGGTGACTCACCACT-3' (SEQ ID NO:9),
5'-TGTGCTGACTCAGCACA-3' (SEQ ID NO:11),
5 '-TTGTGCTGACTCAGCACAA-3' (SEQ ID NO:13),
5'-TGGTGAGTCACCA-3' (SEQ ID NO:15),
5'-ATGGTGAGTCACCAT-3' (SEQ ID NO:17),
5'-TATGGTGAGTCACCATA-3' (SEQ ID NO:19),
5'-CTATGGTGAGTCACCATAG-3' (SEQ ID NO:21),
5'-CCTATGGTGAGTCACCATAGG-3' (SEQ ID NO:23),
5'-TGTTGAGTCACCA-3' (SEQ ID NO:25),
5'-GTGTTGAGTCACCAC-3' (SEQ ID NO:27),
5'-TGTGTTGAGTCACCACA-3' (SEQ ID NO:29),
5'-CTGTGTTGAGTCACCACAG-3' (SEQ ID NO:31),
5'-ACTGTGTTGAGTCACCACAGT-3' (SEQ ID NO:33),
5'-GTCGCTTAGTGACTAAGCGAC-3' (SEQ ID NO:35),

Sequenz gemäß SEQ ID NO: 3-6, 11-14, 35 und 36 sind aus JP 2001095573 bekannt als Transkriptionsfaktor AP-1 bindenden DNA-Oligonukleotiden zur Herstellung eines Arzneimittels zur Behandlung von Gefässkrankheiten.

Die Erfinder haben überraschenderweise beobachtet, dass Neutralisation des Transkriptionsfaktors AP-1 mit Hilfe entsprechender Decoy-Oligonukleotide innerhalb weniger Stunden zu einem Abfall der CD40-Expression in humanen kultivierten Endothelzellen (Figur 1-4) ebenso wie in nativen Endothelzellen der Ratte (Figur 10) führt. Dieser Effekt trat nach circa 4 Stunden auf und hielt für mindestens 10 Stunden an (Figur 1 und 4). Unerwartet und überraschend war zudem, dass dieser Effekt nahezu gleichzeitig und in weitgehend identischem Ausmaß auf der mRNA- und Proteinebene sichtbar wurde. Decoy-Oligonukleotide, die gegen andere Transkriptionsfaktoren gerichtet sind (z.B. *nuclear factor of activated T-cells,* NFAT), beeinflussten die konstitutive CD40-Expression dagegen nicht (Figur 3). Auch Kontroll-Oligonukleotide, die bis auf eine oder zwei Basen eine mit der AP-1 Konsensus-Kembindungssequenz (SEQ ID NO:1 und 2) identische Sequenz aufweisen, zeigten diesen Effekt nicht (Figur 1 und 2). Weiterhin reichte für die Suppression der CD40-Expression eine zweistündige Exposition der Endothelzellen gegenüber dem AP-1-Decoy-Oligonukleotid aus (Figur 4).

Eine Konsequenz der AP-1-Decoy-Oligonukleotid-vermittelten Reduktion des CD40-Proteingehaltes in den Endothelzellen war eine ausgeprägte Hemmung der CD154-induzierten Neubildung von Interleukin-12 p40 (Figur 5), dem Geschwindigkeits-bestimmenden Schritt in der Synthese von biologisch aktivem Interleukin-12 (Lienenlüke et al. (2000) Eur. J. Immunol. 30, 2864). Auch die CD154-induzierte Expression des *vascular cell adhesion molecule-1* (VCAM-1) war in einem vergleichbaren Umfang reduziert (58% Hemmung), und im Einklang damit die CD154-induzierte verstärkte (und primär VCAM-1 vermittelte) Adhäsion von THP-1 Monozyten an die Endothelzellen (Figur 6).

AP-1 (http://www.cbil.upenn.edu/cgi-bin/tess/tess33?request=FCT-DBRTRV-Accno&key=T00029) gehört zu der circa 46 Mitglieder umfassenden Gruppe der sogenannten *basic region leucine zipper* oder bZIP Transkriptionsfaktoren. In der Regel besteht der aktive Transkriptionsfaktor aus einem Jun/Jun-Homodimer oder einem Jun/Fos-Heterodimer. Sowohl Fos (GenBank Accession Number V01512) wie auch Jun (GenBank Accession Number J04111) müssen dazu im Rahmen der Zellaktivierung über entsprechende Proteinkinasen phosphoryliert werden, wobei die Aktivierung dieser Fos- bzw. Jun-Kinase wiederum von der Aktivität anderer, in dem Signaltransduktionsweg weiter oberhalb gelegener Proteinkinasen abhängt (z.B. Proteinkinase C oder *stress-activated protein kinase,* SEK-1). AP-1 spielt, in der Regel zusammen mit anderen Transkriptionsfaktoren, eine wichtige Rolle bei der Expression einer Vielzahl von immunrelevanten Genen wie z.B. Interleukin-2, Interleukin-4, Interleukin-8, Interferon-γ, MCP-1, MIF und Tumornekrosefaktor-α. Eine Blockade der Aktivität von AP-1 kann demnach beispielsweise mit der Interleukin-2-abhängigen autokrinen Stimulation von T-Zellen und deren klonaler Expansion interferieren.

Um keine generelle Schwächung der spezifischen (zellulären bzw. humoralen) Immunabwehr zu erzeugen, wird das erfindungsgemäße Decoy-Oligonukleotid daher vorzugsweise nicht systemisch sondern lokal appliziert. Die *ex vivo*-Behandlung eines Spenderorgans oder einer Knochenmarkspende vor der Transplantation stellen bevorzugte Indikationen dar.

Hierbei ist von besonderer Wichtigkeit, dass die erfindungsgemäßen Decoy-Oligonukleotide unmittelbar nach Aufnahme in die Zielzellen wirksam werden, wohingegen die Wirksamkeit von Antisense- oder RNA-Interferenz-Oligonukleotiden in erster Linie vom Umsatz des Proteins in der Zelle und damit von dessen Resynthese abhängig ist.

Wird ein erfindungsgemäßes Decoy-Oligonukleotid gegen AP-1 in humanen Endothelzellen verwendet, so wird die Expression von CD40, im Gegensatz zur Verwendung eines entsprechenden Kontroll-Decoy-Oligonukleotids, deutlich um mehr als 50% reduziert. Darüber hinaus hat ein Ausschalten der AP-1-Aktivität eine hochsignifikante Inhibierung der CD154-stimulierten Expression von Interleukin-12 p40 bzw. VCAM-1 zur Folge. Dies führt zu einer deutlichen Abschwächung der Endothelzell-T-Zell- bzw. Endothelzell-Monozyten-Wechselwirkung aber auch der T-Zell-Interaktion mit anderen Antigen-präsentierenden Zellen (z.B. Makrophagen, dendritische Zellen und B-Lymphozyten) im Rahmen der Transplantatabstoßung bzw. GvHD.

Diese Wirkungen eines Decoy-Oligonukleotids gegen AP-1 (SEQ ID NO:3) konnten in einem Modell der akuten Transplantatabstoßung (heterotope Herztransplantation bei Ratten) eindeutig belegt werden. Das entsprechende mutierte Kontroll-Oligonukleotid (SEQ ID NO:47) zeigte dagegen keine therapeutische Wirkung und damit die Spezifität dieses therapeutischen Ansatzes. Noch eindrucksvoller und in diesem Ausmaß unerwartet war die über 60%ige Hemmung der chronischen Transplantatabstoßung in demselben tierexperimentellen Modell durch kurzzeitige Exposition der Koronararterien des Transplantats gegenüber dem AP-1 Cis-Element Decoy vor der Implantation.

Insofern stellt die Verwendung der erfindungsgemäßen doppelsträngigen DNA-Oligonukleotide, auch Decoy-Oligonukleotid oder Cis-Element Decoy genannt, die eine Konsensus-Kembindungsstelle für AP-1 enthalten, das bevorzugte Verfahren zur spezifischen Inhibierung der AP-1-Aktivität dar. Die exogene Zufuhr einer großen Zahl von Transkriptionsfaktor-Bindungsstellen zu einer Zelle, insbesondere in viel höherer Zahl als im Genom vorhanden, erzeugt eine Situation, in der die Mehrzahl eines bestimmten Transkriptionsfaktors spezifisch an das jeweilige Cis-Element Decoy und nicht an seine endogenen Ziel-Bindungsstellen bindet. Dieser Ansatz zur Inhibition der Bindung von Transkriptionsfaktoren an ihre endogene Bindungsstelle wird auch als Squelching bezeichnet. Squelching (oder auch Neutralisation) von Transkriptionsfaktoren unter Verwendung von Cis-Element Decoys wurde erfolgreich eingesetzt, um das Wachstum von Zellen zu inhibieren. Dabei wurden DNA-Fragmente verwendet, die spezifische Bindungsstellen für den Transkriptionsfaktor E2F enthielten (Morishita et al., PNAS, (1995) 92, 5855).

Die Sequenz einer Nukleinsäure, die zur Verhinderung der Bindung des Transkriptionsfaktors AP-1 verwendet wird, ist beispielsweise die Sequenz, an die AP-1 natürlicherweise in der Zelle bindet. AP-1 bindet spezifisch an das Motiv mit der Sequenz 5'-VTGAGTCAS-3' (SEQ ID NO:1), wobei V = A, C oder G und S = C oder G bedeutet. Für eine effektive Bindung von AP-1 kommt es auf die exakte Übereinstimmung mit dieser Sequenz an, wobei der komplementäre Strang 5'-STGACTCAB-3' (SEQ ID NO:2) mit S = C oder G und B= G, C oder T den Transkriptionsfaktor ebenso effizient binden kann. Das Cis-Element Decoy kann ferner größer als die 9-mer Kernbindungssequenz sein und am 5'-Ende und/oder am 3'-Ende verlängert werden. Entsprechende Mutationen im Bereich der Kernbindungssequenz (z.B. 5'-VTGACTCAA-3' oder 5'-VTTACTTAG-3') fuhren zu einem teilweisen oder kompletten Verlust der Bindung von AP-1 an das Decoy-Oligonukleotid (Figur 7).

Darüber hinaus begünstigt eine weitgehend palindromische Sequenz der beiden DNA-Stränge den Transport in die Zielzellen ohne Hilfsmittel. Abgesehen von der grundsätzlichen Voraussetzung, dass die erfindungsgemäßen Decoy-Oligonukleotide den Transkriptionsfaktor AP-1 *in vitro* effektiv neutralisieren, ist es für die therapeutische Wirksamkeit entscheidend, dass das DNA-Molekül schnell und in einem ausreichenden Umfang in die Zielzelle aufgenommen wird. Dies wird veranschaulicht durch die unterschiedlich effektive Neutralisierung von AP-1 durch dieselben Decoy-Oligonukleotide in einem zellfreien System (Figur 7) im Vergleich zu intakten Zellen (Figur 8) bzw. deren Wirkung auf die Genexpression in diesen Zellen (Hemmung der IL-1β stimulierten Expression von MCP-1; Figur 9). Darüber hinaus sollte das erfindungsgemäße Cis-Element Decoy eine bestimmte Länge nicht überschreiten, da dies für den Transport in die Zielzelle limitierend ist. Geeignet ist jedes Decoy-Oligonukleotid mit einer Länge von mindestens 9 bp (Konsensus-Kernbindungssequenz) bis zu einer Länge von etwa 45 bp, vorzugsweise bis zu einer Länge von etwa 27 Basenpaaren, besonders bevorzugt bis zu einer Länge von etwa 23, insbesondere bevorzugt mit einer Länge von 9, 10, 11, 12, 13, 14, 15, 16, 17, 18, 19, 20, 21, 22 oder 23 Basenpaaren.

Da das Cis-Element Decoy eine doppelsträngige Nukleinsäure ist, umfasst das erfindungsgemäße DNA-Oligonukleotid jeweils nicht nur die Sense- oder Forward-Sequenz sondern auch die komplementäre Antisense- oder Reverse-Sequenz. Bevorzugte erfindungsgemäße DNA-Oligonukleotide weisen eine 9-mer-Kernbindungssequenz für AP-1 1 auf, wie sie in SEQ ID NO: 1 enthalten ist. Das Cis-Element Decoy kann jedoch auch eine zur vorstehenden Sequenz abweichende Sequenz aufweisen und länger als ein 9-mer sein. Besonders bevorzugt sind die Sequenzen wie sie in SEQ ID NO:3 bis SEQ ID NO:36 enthalten sind. Diese Aufzählung der bevorzugten Sequenzen ist nicht abschließend. Dem Fachmann ist ersichtlich, dass eine Vielzahl von Sequenzen als Inhibitor für AP-1 verwendet werden können, solange sie die vorstehend aufgeführten Bedingungen der 9-mer Konsensus-Kernbindungssequenz und eine Affinität zu AP-1 aufweisen.

Die Affinität der Bindung einer Nukleinsäuresequenz an AP-1 kann durch die Verwendung des Electrophoretic Mobility Shift Assay (EMSA) (Sambrook et al. (1989) Molecular Cloning. Cold Spring Harbor Laboratory Press; Krzesz et al. (1999) FEBS Lett. 453, 191) bestimmt werden. Dieses Testsystem ist für die Qualitätskontrolle von Nukleinsäuren, die für die Verwendung in dem Verfahren der gegenwärtigen Erfindung gedacht sind, oder die Bestimmungen der optimalen Länge einer Bindmgsstelle geeignet. Sie ist auch für die Identifizierung von anderen Sequenzen, die durch AP-1 gebunden werden, geeignet.

Das Verfahren der vorliegenden Erfindung moduliert die Transkription eines Gens oder von Genen in einer solchen Weise, dass das Gen oder die Gene, z.B. CD40, nicht oder vermindert exprimiert werden. Verminderte oder unterdrückte Expression im Rahmen der gegenwärtigen Erfindung bedeutet, dass die Transkriptionsrate verringert ist im Vergleich zu Zellen, die nicht mit einem erfindungsgemäßen Decoy-Oligonukleotid behandelt werden. Solch eine Verminderung kann beispielsweise durch Northem Blot (Sambrook et al., 1989) oder RT-PCR (Sambrook et al., 1989) bestimmt werden. Typischerweise ist eine solche Verringerung zumindest eine 2-fache, besonders zumindest eine 5-fache, insbesondere zumindest eine 10-fache Verringerung.

Der Verlust von Aktivierung kann beispielsweise erreicht werden, wenn AP-1 bei einem bestimmten Gen als Transkriptionsaktivator wirkt und deshalb Squelching des Aktivators zum Verlust der Expression des Zielgens führt. Ebenso möglich ist aber auch eine indirekte Hemmung durch Modulation der mRNA- (posttranskriptionelle Wirkung) bzw. Proteinstabilität (posttranslationelle Wirkung) des Zielgens. In diesem Fall hätte die Neutralisierung von AP-1 z.B. eine verminderte Expression von Proteinen zur Folge, die den Abbau der mRNA des Zielgens durch RNasen und/oder die proteolytische Degradation des Zielproteins verhindern. Eine derartige Wirkung scheint bei dem beschriebenen Effekt der AP-1 Cis-Element Decoys auf die CD40-Expression in den humanen Endothelzellen beteiligt zu sein.

Oligonukleotide werden in der Regel schnell durch Endo- und Exonukleasen, im besonderen DNasen und RNasen in der Zelle abgebaut. Deshalb können die DNA-Oligonukleotide modifiziert werden, um sie gegen den Abbau zu stabilisieren, so dass über einen längeren Zeitraum eine hohe Konzentration der Oligonukeotide in der Zelle beibehalten wird. Typischerweise kann eine solche Stabilisierung durch die Einführung von einer oder mehrerer modifizierter Internukleotidbindungen erhalten werden.

Ein erfolgreich stabilisiertes DNA-Oligonukleotid enthält nicht notwendigerweise eine Modifikation an jeder Internukleotidbindung. Vorzugsweise sind die Internukleotidbindungen an den jeweiligen Enden beider Oligonukleotide des Cis-Element Decoys modifiziert. Dabei können die letzten sechs, fünf, vier, drei, zwei oder die letzte oder eine oder mehrere Internukleotidbindungen innerhalb der letzten sechs Internukleotidbindungen modifiziert sein. Ferner können verschiedene Modifikationen der Internukleotidbindungen in die Nukleinsäure eingefiihrt werden und die daraus entstehenden Decoy-Oligonukleotide auf sequenzspezifische Bindung an AP-1, unter Verwendung des Routine EMSA-Testsystems, getestet werden. Dieses Testsystem erlaubt die Bestimmung der Bindungskonstante des Cis-Element Decoys und so die Bestimmung, ob die Affinität durch die Modifikation verändert wurde. Modifizierte Cis-Element Decoys, die noch eine ausreichende Bindung zeigen, können ausgewählt werden, wobei eine ausreichende Bindung zumindest etwa 50% oder zumindest etwa 75%, und besonders bevorzugt etwa 100% der Bindung der unmodifizierten Nukleinsäure bedeutet.

Cis-Element Decoys mit modifizierter Intemukleotidbindung, die immer noch ausreichende Bindung zeigen, können überprüft werden, ob sie stabiler in der Zelle sind als unmodifizierte Cis-Element Decoys. Die mit den erfindungsgemäßen Cis-Element Decoys "transfizierten" Zellen werden zu verschiedenen Zeitpunkten auf die Menge der dann noch vorhandenen Cis-Element Decoys untersucht. Dabei wird vorzugsweise ein mit einem Fluoreszenzfarbstoff (z.B. Texas-Rot) markiertes Cis-Element Decoy oder ein radioaktiv markiertes (z.B. ³²P oder ³⁵S) Cis-Element Decoy eingesetzt mit anschließender digitaler Fluoreszenzmikroskopie bzw. Autoradiographie oder Szintigraphie. Ein erfolgreich modifiziertes Cis-Element Decoy hat eine Halbwertzeit in der Zelle, die höher ist als die eines unmodifizierten Cis-Element Decoys, vorzugsweise von zumindest etwa 48 Stunden, mehr bevorzugt von zumindest etwa 4 Tagen, am meisten bevorzugt von mindestens etwa 7 Tagen.

Geeignete modifizierte Internukleotidbindungen sind in Uhlmann und Peyman ((1990) Chem. Rev. 90, 544) zusammengefasst. Modifizierte Internukleotid-Phosphat-Reste und/oder Nicht-Phosphor-Brücken in einer Nukleinsäure, die in einem Verfahren der gegenwärtigen Erfindung eingesetzt werden können, enthalten zum Beispiel Methylphosphonat, Phosphorothioat, Phosphorodithioat, Phosphoramidat, Phosphatester, während Nicht-Phosphor-Intemukleotid-Analoge, beispielsweise Siloxan-Brücken, Carbonat-Brücken, Carboxymethylester-Brücken, Acetamidat-Brücken und/oder Thioether-Brücken enthalten. Bei der Verwendung Phosphorothioat-modifizierter Internukleotidbindungen sollten diese vorzugsweise nicht zwischen den Basen Cytosin und Guanin liegen, da dies zu einer Aktivierung der Zielzellen des Cis-Element Decoys fiihren kann.

Eine weitere Ausführungsform der Erfindung ist die Stabilisierung von Nukleinsäuren durch die Einführung struktureller Merkmale in die Nukleinsäure, welche die Halbwertzeit der Nukleinsäure erhöhen. Solche Strukturen, die Haarnadel- und Glocken-DNA enthalten, sind in US 5,683,985 offenbart. Gleichzeitig können modifizierte Internukleotid-Phosphat-Reste und/oder Nicht-Phosphor-Brücken, zusammen mit den genannten Strukturen, eingeführt werden. Die sich daraus ergebenden Nukleinsäuren können im oben beschriebenen Testsystem auf Bindung und Stabilität geprüft werden.

Ein Cis-Element Decoy der vorliegenden Erfindung wird schnell in die Zelle aufgenommen. Eine ausreichende Aufnahme ist durch die Modulation der Expression von einem oder mehreren Genen, die einer Kontrolle durch AP-1 unterliegen, charakterisiert (z.B. CD40). Das Cis-Element Decoy der vorliegenden Erfindung moduliert in bevorzugter Weise die Transkription von einem Gen oder Genen nach etwa 4 Stunden der Berührung mit der Zelle, mehr bevorzugt nach etwa 2 Stunden, nach etwa 1 Stunde, nach etwa 30 Minuten und am meisten bevorzugt nach etwa 10 Minuten. Eine typische Mischung, die in so einem Versuch eingesetzt wird, enthält 10 µmol/l Cis-Element Decoy.

Die vorliegende Erfindung betrifft ferner die Verwendung der erfindungsgemäßen Decoy-Oligonukleotide zur Herstellung eines Arzneimittels, insbesondere zur Prävention und/oder Therapie der akuten und chronischen Transplantatabstoßung, der akuten und chronischen *graft-ersus-host disease* (GvHD) sowie dem Ischämie/Reperfusionsschaden von Organen im Anschluss an einen chirurgischen Eingriff.

Die vorliegende Erfindung betrifft ferner ein Verfahren zur Modulation der Transkription von mindestens einem Gen in Zellen, insbesondere in Endothelzellen und Antigen-präsentierenden Zellen (Monozyten, Makrophagen, dendritische Zellen, B-Zellen), wobei das Verfahren den Schritt der Kontaktierung der genannten Zellen mit einer Mischung, enthaltend eine oder mehrere erfindungsgemäße doppelsträngige Nukleinsäure(n), die in der Lage sind, sequenzspezifisch an den Transkriptionsfaktor AP-1 zu binden, umfasst. Ein bevorzugtes Verfahren ist z.B. die *ex vivo* Behandlung eines Spenderorgans vor Einbringen in den Körper des Empfängers, indem die Nukleinsäure-haltige Mischung in die Blutgefäße des Spenderorgans (orthograd oder retrograd) appliziert wird.

Die Mischung enthaltend die erfindungsgemäßen Cis-Element Decoys wird mit den Zielzellen (z.B. Endothelzellen, Epithelzellen, Leukozyten, glatte Muskelzellen, Keratinozyten oder Fibroblasten) in Berührung gebracht. Das Ziel dieses In-Berührung-Bringens ist die Übertragung der Cis-Element Decoys, die AP-1 binden, in die Zielzelle (d.h. zum Beispiel die AP-1-abhängig CD40-exprimierende Zelle). Deshalb können Nukleinsäure-Modifikation und/oder Zusatzstoffe oder Hilfsstoffe, von denen bekannt ist, dass sie die Durchdringung von Membranen erhöhen, im Rahmen der gegenwärtigen Erfindung benutzt werden (Uhlmann und Peyman (1990) Chem. Rev.90,544).

Eine Mischung gemäß der Erfindung enthält in einer bevorzugten Ausführungsform im wesentlichen nur Nukleinsäure und Puffer. Eine geeignete Konzentration der Cis-Element Decoys liegt im Bereich von zumindest 0,1 bis 100 µM, vorzugsweise bei 10 µM, wobei ein oder mehrere geeignete Puffer zugesetzt werden. Ein Beispiel eines solchen Puffers ist eine modifizierte Ringer-Lösung enthaltend 145 mmol/l Na⁺, 5 mmol/l K⁺, 50 mmol/l Cl⁻, 2 mmol/l Ca²⁺, 1mmol/l Mg²⁺, 10 mmol/l Hepes, 106 mmol/l Isethionat, 10 mmol/l D-Glucose, pH 7,4.

In einer weiteren Ausführungsform der Erfindung enthält die Mischung zusätzlich mindestens einen Zusatzstoff und/oder Hilfsstoff. Zusatzstoffe und/oder Hilfsstoffe wie Lipide, kationische Lipide, Polymere, Liposomen, Nanopartikel, Nukleinsäure-Aptamere, Peptide und Proteine, die an DNA gebunden sind, oder synthetische Peptid-DNA-Moleküle sind beabsichtigt, um beispielsweise die Einbringung von Nukleinsäuren in die Zelle zu erhöhen, um die Mischung auf nur eine Untergruppe von Zellen zu richten, um den Abbau der Nukleinsäure in der Zelle zu verhindern, um die Lagerung der Nukleinsäuremischung vor der Verwendung zu erleichtern. Beispiele für Peptide und Proteine oder synthetische Peptid-DNA-Moleküle sind z.B. Antikörper, Antikörperfragmente, Liganden, Adhäsionsmoleküle, die alle modifiziert oder unmodifiziert sein können.

Zusatzstoffe, die die Cis-Element Decoys in der Zelle stabilisieren, sind beispielsweise Nukleinsäure-kondensierende Substanzen wie kationische Polymere, Poly-L-Lysin oder Polyethylenimin.

Die Mischung, die in dem Verfahren der gegenwärtigen Erfindung eingesetzt wird, wird bevorzugt lokal angewendet durch Injektion, Infusion, Katheter, pluronische Gele, Polymere, die anhaltend Medikamente freisetzen, oder jede andere Vorrichtung, die lokalen Zugang ermöglicht. Auch die *ex vivo* Anwendung der Mischung (Infusion bzw. Inkubation), verwendet im Verfahren der gegenwärtigen Erfindung, erlaubt einen lokalen Zugang.

Die folgenden Figuren und Beispiele dienen nur der Erläuterung und beschränken in keiner Weise den Umfang der Erfindung.

### 1. Zellkultur

Humane Endothelzellen wurden durch Behandlung mit 1,6 U/ml Dispase in Hepes-modifizierter Tyrodelösung für 30 Min. bei 37°C aus Nabelschnurvenen isoliert und auf Gelatine-beschichteten 6-Loch-Gewebekulturschalen (2 mg/ml Gelatine in 0,1 M HC1 für 30 Min. bei Umgebungstemperatur) in 1,5 ml M199 Medium (Gibco Life Technologies, Karlsruhe, Deutschland), enthaltend 20% fötales Kälberserum, 50 U/ml Penicillin, 50 µg/ml Streptomycin, 10 U/ml Nystatin, 5 mM HEPES und 5 mM TES, 1 µg/ml Heparin und 40 µg/ml endothelialer Wachstumsfaktor, kultiviert. Sie wurden durch ihre typische Pflasterstein-Morphologie, positive Immunfärbung für von Willebrandt-Faktor (vWF) und fluorimetrischen Nachweis (FACS) von PECAM-1 (CD31) sowie negative Immunfärbung für glattmuskuläres α-Actin (Krzesz et al. (1999) FEBS Lett. 453, 191) identifiziert.
Die humane Monozyten-Zelllinie THP-1 (ATCC TIB 202), die humane Jurkat-Zelllinie D1.1 (ATCC CRL-10915) sowie die Maus-Myelomzelllinie P3xTB.A7 wurden in RPMI 1640 Medium (Life Technologies), enthaltend 10% fötales Kälbersenim, 50 U/ml Penicillin, 50 µg/ml Streptomycin und 10 U/ml Nystatin, kultiviert.

### 2. RT-PCR-Analyse

Die endotheliale Gesamt-RNA wurde mit dem Qiagen RNeasy Kit (Qiagen, Hilden, Deutschland) isoliert, daran anschließend wurde eine cDNA-Synthese mit einem Maximum von 3 µg RNA und 200 U Superscript™ II Reversetranskriptase (Life Technologies) in einem Gesamtvolumen von 20 µl entsprechend der Herstelleranleitung durchgeführt. Für den Abgleich der cDNA-Beladung wurden 5 µl (ungefähr 75 ng cDNA) der resultierenden cDNA-Lösung und dem Primerpaar (Gibco) für die Elongationsfaktor-1 (EF-1)-PCR mit 1 U Taq DNA-Polymerase (Gibco) in einem Gesamtvolumen von 50 µl benutzt. EF-1 diente als interner Standard für die PCR. Die PCR-Produkte wurden auf 1,5% Agarose-Gelen enthaltend 0,1% Ethidiumbromid separiert und die Intensität der Banden wurde densitometrisch mit einem CCD-Kamerasystem und der One-Dscan Gelanalyse-Software von Scanalytics (Billerica, MA, USA) bestimmt, um in nachfolgenden PCR-Analysen das Volumen der cDNA anzupassen.

Alle PCR-Reaktionen wurden einzeln für jedes Primer-Paar in einem Hybaid OmnE Thermocycler (AWG, Heidelberg, Deutschland) durchgeführt. Die einzelnen PCR-Bedingungen für die cDNA von humanen Endothelzellen waren wie folgt: CD40 (Produktgröße 381 bp, 25 Zyklen, Anlagerungstemperatur 60°C, (Vorwärtsprimer) 5'-CAGAGTTCACTGAAACGGAATGCC-3' (SEQ ID NO:37), (umgekehrter Primer) 5'-TGCCTGCCTGTTGCACAACC-3'(SEQ ID NO:38); EF-1 (Produktgröße 220 bp, 22 Zyklen, Anlagerungstemperatur 55°C, (Vorwärtsprimer) 5'-TCTTAATCAGTGGTGGAAG-3'(SEQ ID NO:39), (umgekehrter Primer) 5'-TTTGGTCAAGTTGTTTCC-3'(SEQ ID NO:40); IL-12p40 (Produktgröße 281 bp, 30 Zyklen, Anlagerungstemperatur 62°C, (Vorwärtsprimer) 5'-GTACTCCACATTCCTACTTCTC-3°(SEQ ID NO:41), (umgekehrter Primer) 5'-TTTGGGTCTATTCCGTTGTGTC-3'(SEQ ID NO:42); MCP-1 (Produktgröße 330 bp, 22 Zyklen, Anlagerungstemperatur 63°C, (Vorwärtsprimer) 5'-GCGGATCCCCTCCAGCATGAAAGTCTCT-3'(SEQ ID NO:43), (umgekehrter Primer) 5'-ACGAATTCTTCTTGGGTTGTGGAGTGAG-3'(SEQ ID NO:44); VCAM-1 (Produktgröße 523 bp, 26 Zyklen, Anlagerungstemperatur 63°C), (Vorwärtsprimer) 5'-CATGACCTGTTCCAGCGAGG-3'(SEQ ID NO:45), (umgekehrter Primer) 5'-CATTCA-CGAGGCCACCACTC-3'(SEQ ID NO:46).

### 3. Electrophoretic Mobility Shift Assay (EMSA)

Die nukleären Extrakte und [³²P]-markierten doppelsträngigen Konsensus-Oligonukleotide (Santa Cruz Biotechnologie, Heidelberg, Deutschland), nicht-denaturierende Polyacrylamid-Gelelektrophorese, Autoradiographie und Supershift-Analyse wurden wie bei Krzesz et al. (1999) FEBS Lett. 453, 191 beschrieben durchgeführt. Dabei wurde ein doppelsträngiges DNA-Oligonukleotid mit der folgenden einzelsträngigen Sequenz verwendet (Kernbindungssequenz ist unterstrichen): AP-1, 5'-CGCTTGATGACTCAGCCGGAA-3' (SEQ ID NO:3). Für die Analyse der Verdrängung von endogenem AP-1 in Kernextrakten der Endothelzellen durch die verschiedenen Cis-Element Decoys wurde im EMSA-Bindungsansatz ein Verhältnis von 50:1 AP-1 Cis-Element Decoy:[³²-P]-markiertes AP-1 Oligonukleotid (11 fmol)) gewählt.

### 4. Decoy-Oligonukleotid-Technik

Doppelsträngige Decoy-Oligonukleotide wurden von den komplementären einzelsträngigen Phosphorothioat-verbundenen Oligonukleotiden (Eurogentec, Köln, Deutschland) wie bei Krzesz et al. (1999) FEBS Lett. 453, 191 beschrieben hergestellt. Die kultivierten humanen Endothelzellen wurden für mindestens 2 Stunden mit dem jeweiligen Decoy-Oligonukleotid in einer Konzentration von 10 µM inkubiert. Im Anschluss daran wurde das Decoy-Oligonukleotidhaltige Medium in der Regel durch frisches Medium ersetzt. Die einzelsträngigen Sequenzen der Oligonukleotide waren wie folgt (unterstrichene Buchstaben kennzeichnen Phosphorothioatverbundene Basen):
- AP-1: 5'-CGCTTGATGACTCAGCCGGAA-3' (SEQ ID NO:3)
- AP-1: 5'-GTGCTGACTCAGCAC-3' (SEQ ID NO:5)
- AP-1: 5'-TGTGCTGACTCAGCACA-3' (SEQ ID NO:11)
- AP-1: 5'-TTGTGCTGACTCAGCACAA-3' (SEQ ID NO:13)
- AP-1: 5'-GTCGCTTAGTGACTAAGCGAC-3' (SEQ ID NO:35)
- AP-1 mut: 5'-CGCTTGATTACTTAGCCGGAA-3' (SEQ ID NO:47)
- NFAT: 5'-CGCCCAAAGAGGAAAATTTGTTTCATA-3' (SEQ ID NO:48)

### 5. Western Blot-Analyse

Die humanen Nabelschnurvenen-Endothelzellen wurden durch fünfmaliges aufeinanderfolgendes Einfrieren in flüssigem Stickstoff und Auftauen bei 37°C aufgeschlossen. Protein-Extrakte wurden wie bei Hecker et al. (1994) Biochem J. 299, 247 beschrieben hergestellt. 20-30 µg Protein wurden mit Hilfe einer 10%igen Polyacrylamid-Gelelektrophorese unter denaturierenden Bedingungen in der Gegenwart von SDS nach Standardprotokoll aufgetrennt und auf eine BioTrace™ Polyvinylidene Fluoride Transfermembran (Pall Corporation, Roßdorf, Deutschland) transferiert. Zum immulologischen Nachweis des CD40-Proteins wurde ein polyklonaler primärer Anti-Human-CD40-Antikörper von Research Diagnostics Inc., Flanders NJ, USA verwendet. Die Proteinbanden wurden nach Hinzufügen eines Peroxidase-gekoppelten-Anti-Kaninchen-IgG (1:3000, Sigma, Deisenhofen, Deutschland) mit Hilfe der Chemilumineszenz-Methode (SuperSignal Chemiluminescent Substrate; Pierce Chemical, Rockford, IL, USA) und nachfolgender Autoradiographie (Hyperfilm™ MP, Amersham Pharmacia Biotech, Buckinghamshire, England) nachgewiesen. Der Auftrag und Transfer gleicher Proteinmengen wurde nach "Strippen" der Transfermembran (5 Minuten 0.2 N NaOH, nachfolgend 3 x 10 Minuten Waschen mit H₂O) durch Nachweis gleicher Proteinbanden von β-Actin mit einem monoklonalen primären Antikörper und einem Peroxidase-gekopplelten Anti-Maus IgG (beide von Sigma-Aldrich, 1:3000 Verdünnung) gezeigt.

### 6. Endothelzellen-Leukozyten-Wechselwirkung

Primär kultivierte humane Endothelzellen, auf Deckgläsern bis zu einer Zelldichte von 100% gewachsen, wurden mit Hepes-Tyrode-Puffer (Angaben in mmol/L: NaCl 137, KCl 2.7, CaCl₂ 1.4, MgCl₂ 0.25, NaH₂PO₄ 0.4, Na-Hepes 10, D-Glucose 5), der 1.5% Polyvinylpyrrolidon (PVP; Sigma-Aldrich) enthielt, gewaschen und auf dem Boden einer Perfusionskammer (2.5 mm Höhe und 260 µl Volumen, Warner Instrument, Hamden, CT, USA) aufgebracht. Die Kammer wurde auf einem Axiovert S100 TV Mikroskop (Zeiss, Goettingen, Germany) auf einer beheizbaren Plattform (Warner Instruments) befestigt und mit dem beschriebenen, auf 37°C erwärmten Puffer *(in-line solution heater,* Warner Instruments) perfundiert. Die mit Hilfe einer Pumpe (Ismatec, Zürich, Schweiz) erzeugte Schubspannung betrug 5 dyn/cm² bei einer Scherrate von 10 S⁻¹. Die Endothelzellen wurden zu Beginn 10 min mit Hepes-Tyrode/PVP perfimdiert, gefolgt von einer 10-minütigen Superfusion mit 1.5x10⁶ THP-1 Zellen (5x10⁵ THP-1 Zellen/ml) in Hepes-Tyrode/PVP. Anschließend wurde die Perfusionskammer mit Hepes-Tyrode/PVP gespült. Die Dokumentation der Zell-Zell-Wechselwirkungen erfolgte bei 20facher Vergrößerung mit einer SPOT RT Farb-CCD Kamera (Diagnostic Instruments, Burroughs St. Sterling Heights, Michigan, USA). Pro Testansatz wurden drei Aufnahmen verschiedener Gesichtsfelder mit dem Programm MetaMorph V3.0 (Universal Imaging, West Chester, PA, USA) ausgewertet.

### 7. Statistische Analyse

Wenn nicht anders angezeigt, sind alle Daten in den Figuren als Mittelwert ± SEM von n Experimenten angegeben. Die statistische Auswertung wurde mittels einseitiger Varianzanalyse (ANOVA) gefolgt von einem Dunnett Post-Test durchgeführt. Ein P-Wert <0,05 wurde als statistisch signifikanter Unterschied angesehen.

### 8. Tierexperimenteller Nachweis der Decoy-Oligonukleotid-Wirkung

Zum Nachweis der Wirksamkeit des in der vorliegenden Anmeldung entwickelten Decoy-Oligonukleotid-basierten Therapieansatzes wurde für die Indikation akute Transplantatabstoßung eine tierexperimentelle *Proof-of-concept*-Studie mit Ratten durchgeführt (Stammkombination Wistar Furth auf Lewis; experimentelle Details siehe Hölschermann et al. (1999) Am. J. Pathol. 154, 211). Einmalige Applikation von 10 µmol/l des AP-1-Decoy-Oligonukleotids (SEQ ID NO:3), nicht aber des mutierten Kontroll-Oligonukleotides (AP-1 mut (SEQ ID NO:47), kein Unterschied im Vergleich zu den Kontrolltieren), in die Koronargefäße des heterotopen Herztransplantates (30 Minuten Inkubation vor der Implantation) verlängerte dessen Überleben ohne Gabe eines Immunsuppressivums von 6,2 ± 0,2 auf 7,6 ± 0,4 Tage (n=5, P<0,05). Dieser Effekt war assoziiert mit einer signifikanten Abschwächung der Adhäsionsmolekülexpression (z.B. VCAM-1) im Endothel der Koronargefäße der Spenderherzen sowie der Infilatration von Monozyten und T-Zellen am postoperativen Tag 1 und 3.

Auch im Modell der Transplantatvaskulopathie (chronische Abstoßung) erwies sich die einmalige Applikation des AP-1 Decoy-Oligonukleotids als äußerst wirksam. In Abwandlung des vorab beschriebenen, akuten Abstoßungsmodells wurden die Empfängertiere mit dem Immunsuppressivum Cyclosporin A (5 mg pro kg Körpergewicht und Tag) intraperitoneal behandelt und die Spenderherzen nach 100 Tagen explantiert. Der Grad der Vaskulopathie in den Koronararterien wurde gemäß den Adams-Kriterien morphometrisch bestimmt und ergab folgendes Bild: Isotyp-Kontrolle (n=7), Score 0,97 ± 0,11; Cyclosporin A-Kontrolle (n=6), Score 2,08 ± 0,16 (P>0,001 versus Isotyp-Kontrolle); AP-1 Decoy-Oligonukleotid (n=6), Score 1,39 ± 0,16 (P<0,01 versus Cyclosporin A-Kontrolle).

## Patentansprüche

1. Doppelsträngige DNA-Oligonukleotide, wobei einer der beiden DNA-Stränge eine Sequenz gemäß SEQ ID NO:1, 2, 7 bis 10 oder 15 bis 34 und der komplementäre DNA-Strang die dazu komplementäre Sequenz aufweist.

2. Doppelsträngige DNA-Oligonukleotide gemäß Anspruch 1 als Arzneimittel.

3. Verwendung von doppelsträngigen, den Transkriptionsfaktor AP-1 bindenden DNA-Oligonukleotiden und von doppelsträngigen DNA-Oligonukleotiden gemäß SEQ ID NO: 1 bis 36 zur Herstellung eines Arzneimittels zur Prophylaxe und/oder Therapie der akuten und chronischen Transplantatabstoßung, der akuten und chronischen *graft-versus-host disease* (GvHD) sowie dem Ischämie/Reperfusionsschaden von Organen im Anschluss an einen chirurgischen Eingriff.

## Claims

1. Double-strand DNA oligonucleotides, wherein one of the two DNA strands provides a sequence according to SEQ ID NO: 1, 2, 7 to 10 or 15 to 34, and the complementary DNA strand provides the sequence complementary to the these.

2. Double-strand DNA oligonucleotides according to claim 1 as pharmaceutical agents.

3. Use of double-strand, transcription-factor-AP-1-binding DNA oligonucleotides, and of double-strand DNA oligonucleotides according to SEQ ID NO: 1 to 36 for the manufacture of a pharmaceutical agent for the prevention and/or treatment of acute and chronic transplant rejection, acute and chronic graft-versus-host disease (GvHD) and ischaemia/reperfusion damage of organs following a surgical intervention.

## Revendications

1. Oligonucléotide d'ADN double brin, dans lequel l'un des deux brins d'ADN présente une séquence SEQ ID NO:1; 2, 7 à 10 ou 15 à 34 et le brin d'ADN complémentaire présente la séquence complémentaire à celles-ci.

2. Oligonucléotide d'ADN double brin selon la revendication 1 comme médicament.

3. Utilisation des oligonucléotides d'ADN double brin liant le facteur de transcription AP-1 et d'oligonucléotides d'ADN double brin selon les séquences SEQ ID NO:1 à 36 pour la fabrication d'un médicament destiné à la prophylaxie et/ou au traitement du rejet aigu et chronique de greffe, de la réaction du greffon contre l'hôte aiguë et chronique ainsi que des lésions d'ischémie et de reperfusion des organes à la suite d'une intervention chirurgicale.


### SEQUENCE LISTING

<110> Avontec GmbH
<120> Decoy-Oligonukleotid-Hemmung der CD40-Expression
<130> HEC-006 PCT
<140> unknown
   <141> 2003-09-02
<150> 102 40 417.8
   <151> 2002-09-02
<160> 48
<170> PatentIn version 3.1
<210> 1
   <211> 9
   <212> DNA
   <213> synthetic sequence
<220>
   <221> misc_feature
   <222> (1) .. (1)
   <223> V = g or c or a
<220>
   <221> Decoy-Oligonukleotid
   <222> (1) .. (9)
   <223>
<220>
   <221> misc_feature
   <222> (9) .. (9)
   <223> S = g or c
<400> 1
   vtgagtcas 9
<210> 2
   <211> 9
   <212> DNA
   <213> synthetic sequence
<220>
   <221> misc_feature
   <222> (1) .. (1)
   <223> S = g or c
<220>
   <221> Decoy-Oligonukleotid
   <222> (1) .. (9)
   <223>
<220>
   <221> misc_feature
   <222> (9)..(9)
   <223> B = g or t or c
<400> 2
   stgastcab 9
<210> 3
   <211> 21
   <212> DNA
   <213> synthetic sequence
<220>
   <221> Decoy-Oligonukleotid
   <222> (1) .. (21)
   <223>
<400> 3
   cgcttgatga ctcagccgga a 21
<210> 4
   <211> 21
   <212> DNA
   <213> synthetic sequence
<220>
   <221> Decoy-Oligonukleotid
   <222> (1) .. (21)
   <223>
<400> 4
   ttccggctga gtcatcaagc g 21
<210> 5
   <211> 15
   <212> DNA
   <213> synthetic sequence
<220>
   <221> Decoy-Oligonukleotid
   <222> (1) .. (15)
   <223>
<400> 5
   gtgctgactc agcac 15
<210> 6
   <211> 15
   <212> DNA
   <213> synthetic sequence
<220>
   <221> Decoy-Oligonukleotid
   <222> (1)..(15)
   <223>
<400> 6
   gtgctgagtc agcac 15
<210> 7
   <211> 15
   <212> DNA
   <213> synthetic sequence
<220>
   <221> Decoy-Oligonukleotid
   <222> (1) .. (15)
   <223>
<400> 7
   gtggtgactc accac 15
<210> 8
   <211> 15
   <212> DNA
   <213> synthetic sequence
<220>
   <221> Decoy-Oligonukleotid
   <222> (1) .. (15)
   <223>
<400> 8
   gtggtgagtc accac 15
<210> 9
   <211> 17
   <212> DNA
   <213> synthetic sequence
<220>
   <221> Decoy-Oligonukleotid
   <222> (1)..(17)
   <223>
<400> 9
   agtggtgact caccact 17
<210> 10
   <211> 17
   <212> DNA
   <213> synthetic sequence
<220>
   <221> Decoy-Oligonukleotid
   <222> (1) .. (17)
   <223>
<400> 10
   agtggtgagt caccact 17
<210> 11
   <211> 17
   <212> DNA
   <213> synthetic sequence
<220>
   <221> Decoy-Oligonukleotid
   <222> (1) .. (17)
   <223>
<400> 11
   tgtgctgact cagcaca 17
<210> 12
   <211> 17
   <212> DNA
   <213> synthetic sequence
<220>
   <221> Decoy-Oligonukleotid
   <222> (1)..(17)
   <223>
<400> 12
   tgtgctgagt cagcaca 17
<210> 13
   <211> 19
   <212> DNA
   <213> synthetic sequence
<220>
   <221> Decoy-Oligonukleotid
   <222> (1)..(19)
   <223>
<400> 13
   ttgtgctgac tcagcacaa 19
<210> 14
   <211> 19
   <212> DNA
   <213> synthetic sequence
<220>
   <221> Decoy-Oligonukleotid
   <222> (1) .. (19)
   <223>
<400> 14
   ttgtgctgag tcagcacaa 19
<210> 15
   <211> 13
   <212> DNA
   <213> synthetic sequence
<220>
   <221> Decoy-Oligonukleotid
   <222> (1)..(13)
   <223>
<400> 15
   tggtgagtca cca 13
<210> 16
   <211> 13
   <212> DNA
   <213> synthetic sequence
<220>
   <221> Decoy-Oligonukleotid
   <222> (1) .. (13)
   <223>
<400> 16
   tggtgactca cca 13
<210> 17
   <211> 15
   <212> DNA
   <213> synthetic sequence
<220>
   <221> Decoy-Oligonukleotid
   <222> (1) .. (15)
   <223>
<400> 17
   atggtgagtc accat 15
<210> 18
   <211> 15
   <212> DNA
   <213> synthetic sequence
<220>
   <221> Decoy-Oligonukleotid
   <222> (1)..(15)
   <223>
<400> 18
   atggtgactc accat 15
<210> 19
   <211> 17
   <212> DNA
   <213> synthetic sequence
<220>
   <221> Decoy-Oligonukleotid
   <222> (1)..(17)
   <223>
<400> 19
   tatggtgagt caccata 17
<210> 20
   <211> 17
   <212> DNA
   <213> synthetic sequence
<220>
   <221> Decoy-Oligonukleotid
   <222> (1)..(17)
   <223>
<400> 20
   tatggtgact caccata 17
<210> 21
   <211> 19
   <212> DNA
   <213> synthetic sequence
<220>
   <221> Decoy-Oligonukleotid
   <222> (1)..(19)
   <223>
<400> 21
   ctatggtgag tcaccatag 19
<210> 22
   <211> 19
   <212> DNA
   <213> synthetic sequence
<220>
   <221> Decoy-Oligonukleotid
   <222> (1) .. (19)
   <223>
<400> 22
   ctatggtgac tcaccatag 19
<210> 23
   <211> 21
   <212> DNA
   <213> synthetic sequence
<220>
   <221> Decoy-Oligonukleotid
   <222> (1) .. (21)
   <223>
<400> 23
   cctatggtga gtcaccatag g 21
<210> 24
   <211> 21
   <212> DNA
   <213> synthetic sequence
<220>
   <221> Decoy-Oligonukleotid
   <222> (1) .. (21)
   <223>
400> 24
<210> 25
   <211> 13
   <212> DNA
   <213> synthetic sequence
<220>
   <221> Decoy-Oligonukleotid
   <222> (1)..(13)
   <223>
<400> 25
   tgttgagtca cca 13
<210> 26
   <211> 13
   <212> DNA
   <213> synthetic sequence
<220>
   <221> Decoy-Oligonukleotid
   <222> (1) .. (13)
   <223>
<400> 26
   tggtgactca aca 13
<210> 27
   <211> 15
   <212> DNA
   <213> synthetic sequence
<220>
   <221> Decoy-Oligonukleotid
   <222> (1) .. (15)
   <223>
<400> 27
   gtgttgagtc accac 15
<210> 28
   <211> 15
   <212> DNA
   <213> synthetic sequence
<220>
   <221> Decoy-Oligonukleotid
   <222> (1) .. (15)
   <223>
<400> 28
   gtggtgactc aacac 15
<210> 29
   <211> 17
   <212> DNA
   <213> synthetic sequence
<220>
   <221> Decoy-Oligonukleotid
   <222> (1)..(17)
   <223>
<400> 29
   tgtgttgagt caccaca 17
<210> 30
   <211> 17
   <212> DNA
   <213> synthetic sequence
<220>
   <221> Decoy-Oligonukleotid
   <222> (1) .. (17)
   <223>
<400> 30
   tgtggtgact caacaca 17
<210> 31
   <211> 19
   <212> DNA
   <213> synthetic sequence
<220>
   <221> Decoy-Oligonukleotid
   <222> (1) .. (19)
   <223>
<400> 31
   ctgtgttgag tcaccacag 19
<210> 32
   <211> 19
   <212> DNA
   <213> synthetic sequence
<220>
   <221> Decoy-Oligonukleotid
   <222> (1) .. (19)
   <223>
<400> 32
   ctgtggtgac tcaacacag 19
<210> 33
   <211> 21
   <212> DNA
   <213> synthetic sequence
<220>
   <221> Decoy-Oligonukleotid
   <222> (1) .. (21)
   <223>
<400> 33
   actgtgttga gtcaccacag t 21
<210> 34
   <211> 21
   <212> DNA
   <213> synthetic sequence
<220>
   <221> Decoy-Oligonukleotid
   <222> (1) .. (21)
   <223>
<400> 34
   actgtggtga ctcaacacag t 21
<210> 35
   <211> 21
   <212> DNA
   <213> synthetic sequence
<220>
   <221> Decoy-Oligonukleotid
   <222> (1) .. (21)
   <223>
<400> 35
   gtcgcttagt gactaagcga c 21
<210> 36
   <211> 21
   <212> DNA
   <213> synthetic sequence
<220>
   <221> Decoy-Oligonukleotid
   <222> (1) .. (21)
   <223>
<400> 36
   gtcgcttagt cactaagcga c 21
<210> 37
   <211> 24
   <212> DNA
   <213> synthetic sequence
<220>
   <221> Primer
   <222> (1) .. (24)
   <223>
<400> 37
   cagagttcac tgaaacggaa tgcc 24
<210> 38
   <211> 20
   <212> DNA
   <213> synthetic sequence
<220>
   <221> Primer
   <222> (1) .. (20)
   <223>
<400> 38
   tgcctgcctg ttgcacaacc 20
<210> 39
   <211> 19
   <212> DNA
   <213> synthetic sequence
<220>
   <221> Primer
   <222> (1)..(19)
   <223>
<400> 39
   tcttaatcag tggtggaag 19
<210> 40
   <211> 18
   <212> DNA
   <213> synthetic sequence
<220>
   <221> Primer
   <222> (1) .. (18)
   <223>
<400> 40
   tttggtcaag ttgtttcc 18
<210> 41
   <211> 22
   <212> DNA
   <213> synthetic sequence
<220>
   <221> Primer
   <222> (1) .. (22)
   <223>
<400> 41
   gtactccaca ttcctacttc tc 22
<210> 42
   <211> 22
   <212> DNA
   <213> synthetic sequence
<220>
   <221> Primer
   <222> (1) .. (22)
   <223>
<400> 42
   tttgggtcta ttccgttgtg tc 22
<210> 43
   <211> 28
   <212> DNA
   <213> synthetic sequence
<220>
   <221> Primer
   <222> (1)..(28)
   <223>
<400> 43
   gcggatcccc tccagcatga aagtctct 28
<210> 44
   <211> 28
   <212> DNA
   <213> synthetic sequence
<220>
   <221> Primer
   <222> (1) .. (28)
   <223>
<400> 44
   acgaattctt cttgggttgt ggagtgag 28
<210> 45
   <211> 20
   <212> DNA
   <213> synthetic sequence
<220>
   <221> Primer
   <222> (1) .. (20)
   <223>
<400> 45
   catgacctgt tccagcgagg 20
<210> 46
   <211> 20
   <212> DNA
   <213> synthetic sequence
<220>
   <221> Primer
   <222> (1) .. (20)
   <223>
<400> 46
   cattcacgag gccaccactc 20
<210> 47
   <211> 21
   <212> DNA
   <213> synthetic sequence
<220>
   <221> Decoy-Oligonukleotid
   <222> (1) .. (21)
   <223>
<400> 47 cgcttgatta cttagccgga a 21
<210> 48
   <211> 27
   <212> DNA
   <213> synthetic sequence
<220>
   <221> Decoy-Oligonukleotid
   <222> (1) .. (27)
   <223>
<400> 48
   cgcccaaaga ggaaaatttg tttcata 27